# EUROPEAN PATENT APPLICATION

(11) **EP 3 819 637 A1**
(43) Date of publication of application: **12.05.2021**
(21) Application number: 19207491.2
(22) Date of filing: 06.11.2019
(51) Int. Cl.: G01N 33/50, G01N 33/68

(54) **MICROFLUIDIC DROPLET SCREENING METHOD AND SYSTEM**

(71) Applicant: Velabs Therapeutics GmbH, 69117 Heidelberg (DE)
(72) Inventor: Antz, Christoph, 69117 Heidelberg (DE)
(74) Representative: Kuttenkeuler, David

(57) **Abstract**

The invention pertains to a method for performing biological assays in microfluidic system using a plurality of microfluidic droplets having reduced signal heterogeneity. Hence, the inventive method provides a screening system with reduced signal noise. Preferably, the screening method of the invention can be used for the characterization and identification of antigen binding constructs such as antibodies or antibody derivatives. The microfluidic droplets contain a signal inducing particle such as an antigen binding construct expressing cell (e.g. antibody producing cell) and at least two reporter particles or cells, which emit a signal as a consequence of the activity of the signal inducing particle e.g. upon binding of the antigen binding construct.

## Description

### FIELD OF THE INVENTION

The invention pertains to a method for performing biological assays in microfluidic system using a plurality of microfluidic droplets having reduced signal heterogeneity. Hence, the inventive method provides a screening system with reduced signal noise. Preferably, the screening method of the invention can be used for the characterization and identification of antigen binding constructs such as antibodies or antibody derivatives.

### DESCRIPTION

Droplet based microfluidics holds great potential for high throughput screening applications. The encapsulation of single cells into droplets allows screening of cell products such as antibodies at very high throughput, e.g. up to several hundred thousand samples per day. For example, individual antibody-secreting cells (e.g. hybridoma- or B-cells) can be encapsulated into droplets together with a recombinant drug target of interest, such as an enzyme. Addition of a fluorogenic substrate of the enzyme will then reveal which droplets contain a cell releasing antibodies which inhibit enzymatic activity and facilitate the specific sorting of these. However, for many screening applications, it would be very useful to co-encapsulate (in addition to the antibody secreting cell) another reporter cell, rather than a recombinant enzyme. This reporter cell could mediate a fluorescence signal (e.g. expression of a reporter enzyme which can easily be detected in droplets; such as β-Gal) upon the desired effect of an antibody, such as the stimulation or inhibition of cellular pathways upon antibody binding of G protein-coupled receptors (GPCRs). GPCRs are the targets of most best-selling drugs and about 40% of all prescription pharmaceuticals.

One major limiting step in high-throughput screening systems is assay signal normalization. In particular, performing complex cell-biologic assays in a single cell format over thousands of individual and independent experimental compartments, as it is possible with microfluidic approaches, can produce huge signal variability between each individual single compartment. Thus, one continuing problem to be solved is to improve and filter the assay signal from the general noise.

US patent application No. 2010/0092955 A1 discloses a gel microdrop composition which may contain a polymer matrix, an effector particle that releases an effector molecule into the polymer matrix, a first reporter particle that emits a first optically detectable signal and a second reporter particle that emits a second optically detectable signal that is distinguishable from the first optically detectable signal, where the effector particle and said first and second reporter particles are encapsulated by the polymer matrix. Methods of screening that employ the gel microdrop composition and methods of making the gel microdrop composition are also disclosed.

WO 2016/174229 discloses a normalization system for high-throughput microfluidic assay systems where a reporter cell and a test cell are combined, and the reporter cell comprises a known first detectable signal and a assay-dependent second detectable signal, in which the normalization of both signals with each other allows to eliminate signal noise due to the three-dimensional positioning of the cells within a microfluidic compartment.

Thus, it is an objection of the invention to further reduce signal noise in a microfluidic assay system to improve the statistical relevance of the results of high-throughput assay approaches, for example antibody detection and analysis.

### BRIEF DESCRIPTION OF THE INVENTION

Generally, and by way of brief description, the main aspects of the present invention can be described as follows:
In **a first aspect,** the invention pertains to a method of screening a plurality of microfluidic droplets with reduced signal heterogeneity, comprising the step of screening at least one microfluidic droplet of the plurality of microfluidic droplets for the presence of a first detectable signal, wherein the at least one microfluidic droplet comprises two or more reporter particles capable of emitting the first detectable signal.
In **a second aspect,** the invention pertains to a microfluidic droplet, wherein the microfluidic droplet is a microfluidic droplet as defined according to the method of the first aspect.
In **a third aspect,** the invention pertains to a method for screening for an antigen binding construct having a biological activity, comprising a step of encapsulating each of a multiplicity of individual candidate antigen binding construct species, or each of a multiplicity of individual particles producing a candidate antigen binding construct species, into a microfluidic droplet in order to obtain a multiplicity of microfluidic droplets of which each comprises not more than one candidate antigen binding construct species, or not more than one individual particle producing a candidate antigen binding construct species, wherein the microfluidic droplet further comprises at least two reporter particles capable of emitting a first detectable signal representative for a biological activity (such as a signalling event), and wherein said at least two reporter particles emit said first detectable signal upon direct or indirect interaction with the candidate antigen binding construct.

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In **a first aspect,** the invention pertains to a method of screening a plurality of microfluidic droplets with reduced signal heterogeneity, comprising the step of screening at least one microfluidic droplet of the plurality of microfluidic droplets for the presence of a first detectable signal, wherein the at least one microfluidic droplet comprises two or more reporter particles capable of emitting the first detectable signal. Preferably the microfluidic droplet is one droplet out of a multiplicity of microfluidic droplets with reduced signal heterogeneity. More preferably wherein the at least one microfluidic droplet constitutes at least 1%, preferably 5%, more preferably 20%, more preferably 50%, more preferably 75% or higher, most preferably about 95% of microfluidic droplets out of the plurality of microfluidic droplets.

In context of the present invention it was surprisingly found that encapsulating in each, or most, of a multiplicity of to-be screened microfluidic droplets two or more reporter particles tremendously and unexpectedly reduces signal heterogeneity and signal noise of the screening assay in particular in a high throughput format.

The term "microfluidic droplet" in context of the present invention refers to an aqueous microcompartment of a certain size that encapsulates an aqueous liquid. The size of the microfluidic droplet is usually expressed as the diameter of the droplet when in a spherical shape. The diameter is generally between 20 and 400 µm, and preferably between 30 and 350 µm, between 40 and 300 µm, between 40 and 250 µm, between 40 and 200 µm or between 40 and 100 µm (wherein each narrower range is preferred to the foregoing broader ranges and "between" includes the values mentioned). In a preferred embodiment, the diameter of the microfluidic droplet is between 2 and 20 times the diameter of the largest particle (e.g. the any particle disclosed herein) in the droplet, preferably between 3 and 18 times, between 4 and 16 times, or between 5 and 14 times or between 6 and 12 times (wherein each narrower range is preferred to the foregoing broader ranges and "between" includes the values mentioned). Preferably, the diameter of the droplet is defined by both the above absolute and relative parameters. For example, the diameter is: (i) between 20 and 400 µm, between 30 and 350 µm, between 40 and 300 µm, between 40 and 300 µm, between 40 and 250 µm, between 40 and 200 µm or between 40 and 100 µm and between 2 and 20 times the diameter of the largest particle in the droplet; (ii) between 20 and 400 µm, between 30 and 350 µm, between 40 and 300 µm, between 40 and 300 µm, between 40 and 250 µm, between 40 and 200 µm or between 40 and 100 µm and between 4 and 16 times the diameter of the largest in the droplet; or (iii) between 20 and 400 µm, between 30 and 350 µm, between 40 and 300 µm, between 40 and 300 µm, between 40 and 250 µm, between 40 and 200 µm or between 40 and 100 µm and between 6 and 12 times the diameter of the largest in the droplet.

Alternatively, the size of the microfluidic droplet can also be defined by volume. For example, it is usually less than 1 microlitre (µl). Preferably, it is less than 500 nanolitres (nl), less than 250, less than 150, less than 100 or less than 50 nl. In a preferred embodiment, it is between 0.05 and 150 nl, preferably between 0.05 and 125 nl, between 0.05 and 100 nl, between 0.05 and 80 nl, or between 0.05 and 4 nl (wherein each narrower range is preferred to the foregoing broader ranges and "between" includes the values mentioned).

A wide variety of compartmentalization or microencapsulation procedures are available (Benita, S., Ed. (1996). Microencapsulation: methods and industrial applications. Drugs and pharmaceutical sciences. Edited by Swarbrick, J. New York: Marcel Dekker) and may be used to create the microfluidic droplet used in accordance with the present invention. Indeed, more than 200 microencapsulation or compartmentalization methods have been identified in the literature (Finch, C. A. (1993) Encapsulation and controlled release. Spec. Publ.-R. Soc. Chem. 138, 35). These include membrane enveloped aqueous vesicles such as lipid vesicles (liposomes) (New, R. R. C., Ed. (1990). Liposomes: a practical approach. The practical approach series. Edited by Rickwood, D. & Hames, B. D. Oxford: Oxford University Press) and non-ionic surfactant vesicles (van Hal, D. A., Bouwstra, J. A. & Junginger, H. E. (1996). Nonionic surfactant vesicles containing estradiol for topical application. In Microencapsulation: methods and industrial applications (Benita, S., ed.), pp. 329-347. Marcel Dekker, New York.). Preferably, the micro-compartments of the present invention are formed from emulsions; heterogeneous systems of two immiscible liquid phases with one of the phases dispersed in the other as droplets of microscopic size (Becher, P. (1957) Emulsions: theory and practice. Reinhold, New York; Sherman, P. (1968) Emulsion science. Academic Press, London; Lissant, K.J., ed Emulsions and emulsion technology. Surfactant Science New York: Marcel Dekker, 1974; Lissant, K.J., ed. Emulsions and emulsion technology. Surfactant Science New York: Marcel Dekker, 1984). Emulsions may be produced from any suitable combination of immiscible liquids. Preferably the emulsion of the present invention has water (containing a particle and other components) as the phase present in the form of droplets and a hydrophobic, immiscible liquid (preferably an oil) as the surrounding matrix in which these droplets are suspended. Such emulsions are termed 'water-in-oil'. This has the advantage that the aqueous phase is compartmentalized in discrete droplets. The external phase, preferably being hydrophobic oil, generally is inert. The emulsion may be stabilized by addition of one or more surface-active agents (surfactants). These surfactants act at the water/oil interface to prevent (or at least delay) separation of the phases. Many oils and many emulsifiers can be used for the generation of water-in-oil emulsions; a recent compilation listed over 16,000 surfactants, many of which are used as emulsifying agents (Ash, M. and Ash, I. (1993) Handbook of industrial surfactants. Gower, Aldershot). Suitable oils are listed below.

Preferably, the aqueous micro-compartments are created, handled and/or controlled in a microfluidic system. This technology is based on the manipulation of continuous liquid flow through micro-fabricated channels. Actuation of liquid flow is implemented either by external pressure sources, external mechanical pumps, integrated mechanical micropumps, or by combinations of capillary forces and electrokinetic mechanisms. Process monitoring capabilities in continuous-flow systems can be achieved with highly sensitive microfluidic flow sensors based on MEMS technology which offer resolutions down to the nanoliter range.

Microfluidic devices typically consist of networks of channels of approximately ten to a few hundred micrometers in diameter into which small quantities of reagents can be injected in a specific sequence, mixed and incubated for a specified time. Assays can be highly parallelized by generating independent compartments using valves (pinching off specific regions of the channels) or two-phase microfluidics, in which aqueous droplets surrounded by an immiscible oil phase serve as closed vessels. These approaches enable drastically reduced assay volumes (pico - nanoliters) and strongly improved throughput. For example, droplets can be generated at rates of more than 1,000 per second. Furthermore, microfluidic modules for the splitting, fusion and sorting of droplets at similar rates have been developed, thus providing a repertoire of manipulations mimicking classical bench top procedures.

In a preferred embodiment (applying to all aspects herein), the device, in particular the channels, is/are large enough to handle droplets comprising eukaryotic cells. In other words, the device, in particular the channels, is/are large enough to handle droplets of the sizes described herein, in particular 100 µm droplets.

Thus, the microcompartment described herein is also termed "microfluidic droplet", e.g. of a water-in-oil emulsion (Schaerli and Hollfelder, The potential of microfluidic water-in-oil droplets in experimental biology. Mol Biosyst (2009) vol. 5 (12) pp. 1392-404).

The "immiscible liquid" is preferably a hydrophobic liquid, preferably oil. The oil phase should allow the micro-compartments to be stable against coalescence, have a viscosity that is close to that of water, and/or be inert with respect to the biological reagents contained in them. Several oils can be used. Low-viscosity silicone oils swell. Polydimethylsiloxane, also called PDMS or dimethicone. PDMS is a polymer widely used for the manufacture of microfluidic chips, and it is a mineral-organic polymer (a structure containing carbon and silicon of the siloxane family), which can change the cross-sectional dimensions of microfluidic channels and influencing the flow properties of microfluidic devices if swelling occurs. Silicone oils can also be used in microfluidic devices fabricated in glass, which are impermeable to these oils. High-viscosity silicone oils can be used in PDMS devices with minimal swelling at the expense of significantly increasing the pressures required to pump them through the micro-channels. Hydrocarbon oils can also be obtained in a range of viscosities and have the benefit that there are a large number of commercially available surfactants for them that can stabilize aqueous-in-oil emulsions. However, they also swell PDMS and tend to exhibit poor retention of encapsulated organic reagents, which are often partially soluble in these oils. The preferred oils for use in the methods of the invention are fluorocarbon oils (or flourinated oils), because even low-viscosity versions of these oils do not swell PDMS. In addition, they tend to exhibit excellent retention of reagents in the drops and have high solubility for gases, allowing oxygen and carbon dioxide to passively diffuse in and out of the micro-compartments, for unperturbed cellular respiration. This allows cells to survive in fluorocarbon oil emulsions for hours after encapsulation. A disadvantage of fluorocarbon oils, however, is that, due to their much lower prevalence compared with silicone and hydrocarbon oils, there are fewer commercially available surfactants for stabilizing aqueous-in-fluorocarbon emulsions. Surfactants are useful for reducing the surface tension of the oil-water interface and minimizing droplet coalescence. The choice of which surfactant to use is also important for limiting the transfer of reagents between micro-compartments. A comprehensive review of surfactants for droplet-based microfluidics is given in Baret (2012 Lab Chip 12, 422). The surfactants utilized in droplet-based microfluidics normally consist of a hydrophilic head group and hydrophobic tail. The amphiphilic character of these molecules allows them to assemble at the oil-water interface of the droplet, thereby lowering its interfacial tension and enhancing stability. The chemical properties of the head group of the surfactant impact the biocompatibility of the droplet interface. Surfactants with non-ionic head groups are preferred, as they minimize the adsorption of macromolecules such as proteins and DNA to the droplet interface, minimally impacting the methods of the invention. Suitable fluoro-surfactants that can be readily synthesized in the lab are known in the art and described, e.g., in Clausell-Tormos J et al 2008 Chem. Biol. 15, 427-37 or Sadtler et al. 1996 Angew. Chem. Int. Edn Engl. 35, 1976-8. Additives to the aqueous phase can also enhance biocompatibility by increasing the retention of small molecules in the droplets and minimizing adsorption at the oil-water interface. Different oils can be mixed to optimize the properties of the emulsion for a particular application and methods for easily characterizing the properties of the combination that has been selected are known in the art (Kaltenbach et al. 2012 Lab Chip 12, 4185).

The term "particle" refers to any object small enough to fit into the microfluidic droplet and which can be labeled with a detectable label defined herein. Preferably, it is selected from the group consisting of a (biological) cell, a bead, a virus, a protein and a nanoparticle. The cell can be any prokaryotic or eukaryotic cell. Preferably, it is a eukaryotic cell, e.g. a yeast cell, plant cell or animal cell. Animal cells include insect, nematode, fish and mammalian cells. More preferably it is mammalian cell, e.g. a mouse, rat, monkey or human cell. For example, it can be a random cell of a heterogeneous cell population (e.g. from a tissue) or it can be a specifically selected cell, selected, e.g., by FACS. Also, it can be a cell from cell line or a homogeneous culture, for example of a primary cell, wherein "primary" means derived directly from a tissue or organism and not manipulated to have altered properties, e.g. to divide indefinitely. Cells in certain examples are not yeast cells, which are known for their inter-cell genetic heterogeneity. Other examples for cells are developing cells, stem cells or cancer cells. A preferred particle of the invention is a reporter particle, which is to be understood that the particle is capable of inducing or emitting a detectable signal (produced directly or indirectly by a detectable label) upon being contacted with an assay candidate. Such reporter particle therefore "reports" via such detectable signal whether the assay candidate has or does not have a particular biological activity. In preferred embodiments of the present invention the reporter particle is a reporter cell, for example signaling, preferably by fluorescence, the effect of the antibody produced by an antibody-producing cell on the reporter cell (e.g. the activation or inhibition of a receptor, preferably a G-protein coupled receptor).

A "bead" (also termed "microbead") in context of the present invention is a uniform polymer particle with a diameter of up to 1 micrometre, preferably of 0.5 to 500 µm, and with a surface to which biological entities such as cells, proteins including antibodies and/or nucleic acids as well as detectably labels as described below can bind or be coupled. The beads referred to herein are usually polyethylene or polystyrene beads or beads made of gel matrices, for example coated with protein/peptide/antigen, e.g. cellular antigen (e.g. a cell surface antigen).

The term "virus" refers to a small infectious agent replicating only inside living cells of a host organism. Preferably, it is a pathogenic virus, more preferably a virus pathogenic in mammals, in particular humans, e.g. from the class of herpesviridae, adenoviridae, papillomaviridae, polyomaviridae, poxviridae, anelloviridae, parvoviridae, reoviridae, coronaviridae, astroviridae, caliciviridae, flaviviridae, picornaviridae, togaviridae, rhabdoviridae, filoviridae, paramyxoviridae, arenaviridae, bunyaviridae, orthomyxoviridae, retroviridae or hapadnaviridae.

The term "protein" as used herein indicates a polypeptide with a particular secondary and tertiary structure that can interact with another analyte and in particular, with other biomolecules including other proteins, DNA, RNA, lipids, metabolites, hormones, chemokines, and small molecules. In can be a natural (i.e. unaltered by man) or a recombinant protein. Preferably, other than proteins defined above such as antibodies, it is a cell surface-displayed molecule, a receptor or a receptor ligand.

The term "nanoparticle" used herein refers to a particle having a diameter of from about 1 to 1000 nm, preferably 1 to 100 nm. Components of the nanoparticle may include metal such as gold, silver, copper, aluminum, nickel, palladium, platinum, alloys thereof, a semiconductor material such as CdSe, CdS, InAs, InP, or core/shell structures thereof, or organic particles such as particles made from organic polymer, lipids, sugars, or other organic materials, e.g. polystyrene, latex, acrylate, or polypeptide. Such organic particles may optionally contain some inorganic material; however, the amount of inorganic material is less than 50%, less than 25%, less than 10%, less than 5%, or less than 1%.

As used herein, the term "detectable signal" refers to a change in or appearance of a property in the reporter system which is capable of being perceived or sensed, either by direct observation or instrumentally, and which is a function of the presence of the fusion protein(s) in the test sample. Some examples of detectable signals are changes in visible or infra-red absorption fluorescence, phosphorescence or chemiluminescence. Other examples of detectable signals may be directed to a change in electrochemical property. In preferred embodiments a detectable signal is emitted by, or produced by, or producible by, a detectable label.

The term "detectable label" (or "marker" or "tag") as used herein refers to any kind of substance which is able to indicate the presence of another substance or complex of substances, in particular of a particle or a potential particle binding partner as defined herein, when associated with it. The detectable label can be a substance that is linked to or introduced or incorporated into the substance to be detected. Preferred is a detectable label suitable for allowing for detection and optionally also quantification, e.g. a detectable label emitting a detectable and preferably measurable signal, preferably a light signal. Examples of suitable labels include a dye, a fluorophore, a fluorescent nanoparticle (e.g. quantum dot or lipidot), a chromophore, a radiolabel, a metal colloid, an enzyme (e.g. alkaline phosphatase, luciferase, beta-galactosidase or horseradish peroxidase), or a chemiluminescent or bioluminescent molecule. In the context of the present invention, suitable detectable labels are preferably protein tags whose peptide sequences are genetically grafted into or onto a recombinant protein. Protein tags preferably are fluorescence tags. Fluorescence tags are used to give visual readout on a protein. GFP and its variants (e.g. mutant GFPs having a different fluorescent spectrum) and RFP and its variants (e.g. mutant RFPs having a different fluorescent spectrum) are the most commonly used fluorescence tags. More advanced applications of GFP/RFP include using it as a folding reporter (fluorescent if folded, colorless if not). Further examples of fluorophores include fluorescein, rhodamine, and sulfoindocyanine dye Cy5.

As such in preferred embodiments the detectable label is directly associated with the reporter particle of the invention. In context of the present invention a "reporter signal" (or first detectable label) shall further denote a signal or a label producing or mediating such signal, which reports any respective assay activity to be screened for. On the other hand the present invention may in addition refer to a "normalization signal" (second detectable label) which is not dependent on the biological activity screened for, but is a known signal used to normalize against inter-droplet, or inter-cell, variability. For example, a constitutively active and known signal in a cell/particle may be used as a normalization signal to correct for spatial localization of the cell/particle within the microfluidic droplet. Such a method for normalization is disclosed in WO 2016/174229, incorporated herein by reference in its entirety.

In some preferred embodiments of the invention the number of reporter particles comprised in the microfluidic droplet is controlled and does not exceed +/- two, preferably one, reporter particle, preferably the number is exactly controlled.

In preferred embodiments of the invention a microfluidic droplet comprises two to fifteen reporter particles, more preferably two to ten, more preferably two to eight, more preferably two to six, more preferably three to six, even more preferably four to seven, or four to six most preferably three, four, five, or six reporter particles. In context of the invention it has been taken into account that one cannot encapsulate endless numbers of cells within one droplet. To the contrary, the inventive composition of signal inducing particles and reporter particles is controlled in order to obtain a stable signal with is in between individual droplets less heterogeneous as compared to a situation with one single particle.

More preferably, the method of the invention the plurality of microfluidic droplets comprises less than 10% of microfluidic droplets comprising 8 or more, preferably 9 or more, reporter particles. More preferably, the plurality of microfluidic droplets comprises less than 5% of microfluidic droplets comprising 8 or more, preferably 9 or more, reporter particles. Even more preferably, the plurality of microfluidic droplets comprises less than 2% of microfluidic droplets comprising 8 or more, preferably 9 or more, reporter particles. Alternatively, or preferably additionally, the plurality of microfluidic droplets comprises less than 20%, more preferably less than 15%, of microfluidic droplets which comprise less than 2 reporter particles (1 or o reporter particles).

In further preferred embodiments, alternative or additional, the majority (50% or more, 60% or more, more preferably 70% or more) of microfluidic droplets of the plurality of microfluidic droplets comprises 2 to 8 reporter particles. In further preferred embodiments of the invention, the majority (50% or more, 60% or more, more preferably 70% or more) of microfluidic droplets of the plurality of microfluidic droplets comprises 2 to 6 reporter particles (50% or more, 60% or more). In further preferred embodiments of the invention, 40% or more (50% or more, 60% or more, more preferably 70% or more) of microfluidic droplets of the plurality of microfluidic droplets comprises 3 to 6 reporter particles. In further preferred embodiments of the invention, the majority (50% or more, 60% or more) of microfluidic droplets of the plurality of microfluidic droplets comprises 2 to 5 reporter particles.

In one particular embodiment, all reporter particles comprised within the microfluidic droplet are substantially identical or identical. The terms "substantially identical" and "identical" in context of the disclosed invention shall mean that two particles or cells have the same makeup with respect to their associated detectable signals/labels, and respond to the same external biological signals. In particular "substantially identical" or "identical" particles/cells in accordance with the invention comprise a label make up consisting of the identical number of label species (for example fluorescent colour of a certain wavelength being a label species) and which labels emit a signal in response to the same biological activity. Substantially identical shall further in some embodiments pertain to cells which are isogenic, so are derived from the same cell population, for example a cell culture.

In another preferred embodiment of the herein disclosed invention, the at least one microfluidic droplet does not comprise any further reporter particle, the further reporter particle being characterized in that it is non-identical to the two or more reporter particles. According to the invention it is advantageous to reduce signal heterogeneity by using a certain plurality of substantially identical or identical reporter particles within each to be screened microfluidic droplet. Since the number of particles to be encapsulated in a microfluidic droplet according to the invention is limited by the size of the droplet, it is preferred to avoid using multiple substantially non-identical, or non-identical, reporter particles within one droplet.

Furthermore, in preferred embodiments of the invention, the number of reporter particles encapsulated is controlled by encapsulating the target number of particles into each microfluidic compartment. Preferably, such embodiments exclude a step of cell cultivation and cell division (multiplication) of a reporter cell within the microfluidic compartment (droplet) . In other words, the present invention intends to avoid the use of microcolony formation within a microfluidic compartment. By avoiding such microcolony formation, heterogeneity between microfluidic compartments during screening is reduced.

In another embodiment of the invention, the at least one microfluidic droplet comprises one or more candidate signal inducing particles. The term "candidate signal inducing particle" in accordance with the herein disclosed invention shall refer to a second species of particle encapsulated in the at least one microfluidic droplet which is distinct from the aforementioned two or more reporter particles. A candidate signal inducing particle may directly or indirectly interact with the two or more reporter particles such that they may induce the two or more reporter particles to emit their respective reporting signal.

In preferred embodiments of the invention, the at least one microfluidic droplet comprises not more than one single candidate signal inducing particle. Hence, one particular preferred embodiment pertains to a method of screening a plurality of microfluidic droplets with reduced signal heterogeneity, comprising the step of screening at least one microfluidic droplet of the plurality of microfluidic droplets for the presence of a first detectable signal, wherein the at least one microfluidic droplet comprises particles, and the entirety of particles comprised in said at least one microfluidic droplet consists of (i) two or more reporter particles capable of emitting the first detectable signal, and (ii) one single candidate signal inducing particle. Hence, preferred is that said at least one microfluidic droplet does not comprise any further species of particles other than one species of reporter particles and one single candidate signal inducing particle. More preferably the entirety of particles comprised in said at least one microfluidic droplet consists of (i) three or more reporter particles capable of emitting the first detectable signal, and (ii) one single candidate signal inducing particle. More preferably the entirety of particles comprised in said at least one microfluidic droplet consists of (i) two to ten reporter particles of the same reporter particle species which are capable of emitting the first detectable signal, and (ii) one single candidate signal inducing particle. More preferably the entirety of particles comprised in said at least one microfluidic droplet consists of (i) three to six reporter particles of the same reporter particle species which are capable of emitting the first detectable signal, and (ii) one single candidate signal inducing particle.

In preferred embodiments of the present invention a direct or indirect interaction between the one or more candidate signal inducing particles and the two or more reporter particles may induce emitting the first detectable signal by the two or more reporter particles. Preferably a direct interaction is a binding between the one or more candidate signal inducing particle to the two or more reporter particles, and/or wherein an indirect interaction is a release of one or more compounds from the one or more candidate signal inducing particle, and wherein such released compounds directly or indirectly interact with the two or more reporter particles.

In a preferred embodiment of the invention a multiplicity of microfluidic droplets according to the invention is screened. Hence, in preferred embodiments the multiplicity of microfluidic droplets comprise a fraction of microfluidic droplets which comprise non-identical single candidate signal inducing particles. As an illustrative example, such non-identical candidate signal inducing particles may all be B-cells expressing a different antibody species. Hence, in such embodiment screening a multitude of B-cells for an antibody with a desired activity is possible.

In a preferred embodiment the reporter particle is a biological cell which emits the first detectable signal upon a biological, chemical or physical stimulus, preferably wherein the stimulus involves or induces any one or a combination of a protein-protein interaction, receptor-ligand interaction, molecule influx or efflux, etc. Preferably the first detectable signal is emitted upon activation of an intracellular signal transduction pathway within the biological cell.

As mentioned elsewhere herein a particle in context of the invention to be encapsulated in a microfluidic droplet for the inventive screening process is preferably a biological cell which may comprise a signal probe, and said signal probe emits the first detectable signal upon said biological, chemical or physical stimulus. Preferably the first detectable signal may be emitted upon activation and expression of one or more genetic elements, preferably wherein said genetic elements encode for a reporter protein (GFP etc). The term "reporter protein" as used herein refers to fluorescent and non-fluorescent reporter proteins including (without being limited to) green fluorescent proteins (GFP), red fluorescence proteins (RFP), yellow fluorescent proteins (YFP), blue and cyan fluorescent proteins (CFP), luciferase, secreted alkaline phosphatase (SEAP), chloramphenicol acetyltransferase (CAT), secreted hormone, secreted cytokine, β-galactosidase, and other fluorescent and bioluminescent proteins. The method according to the invention thus preferably applies a detectable signal which is a fluorescent or luminescent signal.

Preferably, for conducting the screening of the invention, the first detectable label may be detected qualitatively (present or not present) or more preferably quantitatively. The latter allows for a normalization of signal influences not induced by the candidate signal inducing particle but by mere biological variation or other noise sources.

Preferably in context of the invention, the two or more reporter cells may emit a second detectable signal, wherein the second detectable signal is independent of the first detectable signal, and preferably which is essentially independent of any biological, chemical or physical stimulus within the microfluidic droplet. Such second detectable label is used as a normalization signal as described herein elsewhere.

In a most preferred embodiment, the screening method of the invention is for the detection and analysis of antibodies having a particular function such as binding or inducing a biological signal. In such preferred embodiment the microfluidic droplet comprises a single antibody producing cell, such as a B-cell, and wherein the two or more reporter particles emit the first detectable signal upon an interaction with an antibody produced by said antibody producing cell. A similar embodiment can be envisioned using T-cell receptor expressing cells as candidate signal inducing cells, and MHC-peptide presenting cells as reporter cells according to the various descriptions herein.

As such, the association between the reporter particle(s) and the first detectable signal depends on whether the potential particle binding partner, which is released from or comprised in or associated with the candidate signal inducing particle, binds the reporter particle, and the amount of the first detectable signal associated with the reporter particle depends on the binding of the potential binding partner to the particle as well as the number of binding sites if there is more than one binding site on the reporter particle. Preferably, the binding between the potential particle binding partner and the particle is by non-covalent bonds, e.g. by electrostatic interactions, hydrophobic interactions, hydrogen bonds and/or Van der Waals forces. In a preferred embodiment, the binding between the potential particle binding partner and the particle is specific. "Specific binding" as used herein refers to a binding reaction which is determinative of the presence of the binding partner (e.g. the potential particle binding partner or the particle) for example in a situation in which different particles or potential binding partners, which do not specifically bind, are present. Generally, "specifically binds" refers to an equilibrium dissociation constant KD that is less than about 10-5 M (e.g., 10-6 M, 10-7 M, 10-8 M, 10-9 M, 10-10 M, 10-11 M, and 10-12 M or less), preferably at room temperature or at a temperature suitable for maintaining cells, such as about 34-40°C, 35-39°C, 36-38°C or preferably about 37°C. Methods of how to measure equilibrium dissociation constants are well known in the art. A preferred method uses a BiaCore® system. However, the strength or amount of the first detectable signal is not necessarily dependent on the binding strength of an antigen binding protein. For example, similar strong antibody-antigen interactions may, depending on the bound epitope result in either antagonistic or agonistic signalling activity of the reporter particle. Thus, binding strength or antigen affinity is not necessarily screened for, but might be one out of many characteristics targeted by the screening approach of the present invention.

The term "potential reporter particle binding partner" refers to any type of binding partner associated with, or released by, the candidate signal inducing particle, wherein "potential" means that the particle binding partner may or may not bind the reporter particle(s) within the same microfluidic droplet. Preferably, the particle binding partner is selected from the group consisting of an antibody, an antibody derivative, an antibody mimetic, T cell receptor, or antigen binding fragment thereof, a bacteriophage, an mRNA-polypeptide complex, an mRNA-ribosome-polypeptide complex, a cell, a virus, a peptide, a protein, a nucleic acid, an aptamer and a small molecule.

The term "multiplicity, "population" or "plurality" means "more than one". In preferred embodiments, it means more than 10, more than 20, more than 30, more than 40, more than 50, more than 100, more than 250, more than 500 or more than 1000.

In a **second aspect,** the invention pertains to a microfluidic droplet, wherein the microfluidic droplet is a microfluidic droplet as defined according to the method of the first aspect.

In **a third aspect,** the invention pertains to a method for screening for an antigen binding construct having a biological activity, comprising a step of encapsulating each of a multiplicity of individual candidate antigen binding construct species, or each of a multiplicity of individual particles producing a candidate antigen binding construct species, into a microfluidic droplet in order to obtain a multiplicity of microfluidic droplets of which each comprises not more than one candidate antigen binding construct species, or not more than one individual particle producing a candidate antigen binding construct species, wherein the microfluidic droplet further comprises at least two reporter particles capable of emitting a first detectable signal representative for a biological activity (such as a signalling event), and wherein said at least two reporter particles emit said first detectable signal upon direct or indirect interaction with the candidate antigen binding construct.

The term "antigen binding construct" includes all varieties of antibodies and T cell receptor (TCR) derived polypeptides, which comprise an epitope binding domain, including binding fragments thereof. Further included are constructs that include 1, 2, 3, 4, 5, and/or 6 Comple-mentary Determining Region (CDR)s, the main regions mediating antibody or TCR binding ability and specificity to a given antigenic epitope. In some embodiments, these CDRs can be distributed between their appropriate framework regions in a typical antibody or TCR variable domain. In some embodiments, the CDRs can be within a single peptide chain in others they are located in two or more peptide chains (heavy/light or alpha/beta respectively). In some embodiments, the two or more peptides are covalently linked together, for example via disulfide bonds. In some embodiments, they can be linked via a linking molecule or moiety. In some embodiments, the antigen binding proteins are non- covalent, such as a diabody and a monovalent scFv.

The term "isolated" as used herein in the context of a polypeptide, such as an antigen binding construct (an example of which could be an antibody), refers to a polypeptide that is purified from proteins or polypeptides or other contaminants that would interfere with its therapeutic, diagnostic, prophylactic, research or other use. An antigen binding construct according to the invention may be a recombinant, synthetic or modified (non-natural) antigen binding con-struct. The term "isolated" as used herein in the context of a nucleic acid or cells refers to a nucleic acid or cells that is/are purified from DNA, RNA, proteins or polypeptides or other contaminants (such as other cells) that would interfere with its therapeutic, diagnostic, prophylactic, research or other use, or it refers to a recombinant, synthetic or modified (non-natural) nucleic acid. In this context, a "recombinant" protein/polypeptide or nucleic acid is one made using recombinant techniques. Methods and techniques for the production of recombinant nucleic acids and proteins are well known in the art.

As mentioned earlier, preferred antigen binding constructs are antibodies and antibody-like constructs. The term "antibody" includes, but is not limited to, genetically engineered or oth-erwise modified forms of immunoglobulins, such as intrabodies, chimeric antibodies, fully human antibodies, humanized antibodies (e.g. generated by "CDR-grafting"), antibody fragments, and heteroconjugate antibodies (e.g., bispecific antibodies, diabodies, triabodies, tetra-bodies, etc.). The term "antibody" includes cys-diabodies and minibodies. Thus, each and every embodiment provided herein in regard to "antibodies", or "antibody like constructs" is also envisioned as, bi-specific antibodies, diabodies, scFv fragments, chimeric antibody re-ceptor (CAR) constructs, diabody and/or minibody embodiments, unless explicitly denoted otherwise. The term "antibody" includes a polypeptide of the immunoglobulin family or a polypeptide comprising fragments of an immunoglobulin that is capable of non-covalently, reversibly, and in a specific manner binding a corresponding. An exemplary antibody structural unit comprises a tetramer. In some embodiments, a full length antibody can be composed of two identical pairs of polypeptide chains, each pair having one "light" and one "heavy" chain (connected through a disulfide bond). Antibody structure and isotypes are well known to the skilled artisan (for example from Janeway's Immunobiology, 9th edition, 2016).

The recognized immunoglobulin genes of mammals include the kappa, lambda, alpha, gamma, delta, epsilon, and mu constant region genes, as well as the myriad immunoglobulin variable region genes (for more information on immunoglobulin genes see the international Im-MunoGeneTics information system®, Lefranc M-P et al, Nucleic Acids Res. 2015 Jan;43(Database issue):D413-22; and http://www.imgt.org/). For full-length chains, the light chains are classified as either kappa or lambda. For full-length chains, the heavy chains are classified as gamma, mu, alpha, delta, or epsilon, which in turn define the immunoglobulin classes, IgG, IgM, IgA, IgD, and IgE, respectively. The N-terminus of each chain defines a variable region of about 100 to 110 or more amino acids primarily responsible for antigen recognition. The terms variable light chain (VL) and variable heavy chain (VH) refer to these regions of light and heavy chains respectively. As used in this invention, an "antibody" encompasses all variations of antibody and fragments thereof. Thus, within the scope of this concept are full length antibodies, chimeric antibodies, humanized antibodies, single chain antibodies (scFv), Fab, Fab', and multimeric versions of these fragments (e.g., F(ab')2) with the same, essentially the same or similar binding specificity. Antigen binding constructs according to the invention include an antibody heavy chain, preferably the variable domain thereof, or an antigen binding fragment thereof, and/or an antibody light chain, preferably the variable domain thereof, or an antigen binding fragment thereof.

Alternatively, an antigen binding construct of the invention may be a T-cell receptor (TCR), or an antigen binding fragment thereof, and a fusion protein comprising an antigen binding fragment of the aforesaid, such as a bispecific molecule or other multispecific molecule, a chimeric antigen receptor (CAR) etc.

The method of the third aspect is preferred in some embodiments, wherein if, upon direct or indirect interaction of the candidate antigen binding construct with the two or more reporter particles, the first detectable signal compared to a control is (i) increased, said candidate antigen binding construct has an agonistic biological activity, or is (ii) decreased, said candidate antigen binding construct has an antagonistic biological activity.

In further preferred embodiments the two or more reporter particles are biological cells.

For example, preferably, the microfluidic droplet may comprise two to fifteen reporter particles, more preferably two to ten, more preferably two to eight, more preferably two to six, more preferably three to six. The aforesaid for the first aspect applies with respect to the components and characteristics of the at least one microfluidic droplet also for the third aspect.

The method according to the invention in particular embodiments comprises a further step of sorting the microfluidic droplet according to the result of the screening step.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

In view of the above, it will be appreciated that the present invention also relates to the following itemised embodiments:
**Item 1:** A method of screening a plurality of microfluidic droplets with reduced signal heterogeneity, comprising the step of screening at least one microfluidic droplet of the plurality of microfluidic droplets for the presence of a first detectable signal, wherein the at least one microfluidic droplet comprises two or more reporter particles capable of emitting the first detectable signal.
**Item 2:** The method according to item 1, wherein the at least one microfluidic droplet comprises two to fifteen reporter particles, more preferably two to ten, more preferably two to eight, more preferably two to six, more preferably three to six.
**Item 3:** The method according to item 1 or 2, wherein the two or more reporter particles are identical.
**Item 4:** The method according to item 3, wherein the at least one microfluidic droplet does not comprise any further reporter particle, the further reporter particle being characterized in that it is non-identical to the two or more reporter particles.
**Item 5:** The method according to any one of items 1 to 4, wherein the at least one microfluidic droplet comprises one or more candidate signal inducing particles.
**Item 6:** The method according to item 5, wherein the at least one microfluidic droplet comprises not more than one single candidate signal inducing particle.
**Item 7:** The method according to item 5 or 6, wherein a direct or indirect interaction between the one or more candidate signal inducing particles and the two or more reporter particles may induce emitting the first detectable signal by the two or more reporter particles.
**Item 8:** The method according to item 7, wherein a direct interaction is a binding between the one or more candidate signal inducing particle to the two or more reporter particles, and/or wherein an indirect interaction is a release of one or more compounds from the one or more candidate signal inducing particle, and wherein such compounds directly or indirectly interact with the two or more reporter particles.
**Item 9:** The method according to any one of items 1 to 8, the method comprising screening a multiplicity of microfluidic droplets.
**Item 10:** The method according to item 9, wherein the multiplicity of microfluidic droplets comprise a fraction of microfluidic droplets which comprise non-identical single candidate signal inducing particles.
**Item 11:** The method according to any one of items 1 to 10, wherein the reporter particle is a biological cell which emits the first detectable signal upon a biological, chemical or physical stimulus, preferably wherein the stimulus involves or induces any one or a combination of a protein-protein interaction, receptor-ligand interaction, molecule influx or efflux, etc.
**Item 12:** The method according to any one of items 10 to 12, wherein the first detectable signal is emitted upon activation of an intracellular signal transduction pathway within the biological cell.
**Item 13:** The method according to item 11 or 12, wherein the biological cell comprises a signal probe, and said signal probe emits the first detectable signal upon said biological, chemical or physical stimulus.
**Item 14:** The method according to any one of items 10 to 12, wherein the first detectable signal is emitted upon activation and expression of one or more genetic elements, preferably wherein said genetic elements encode for a reporter protein (GFP etc).
**Item 15:** The method according to any one of items 1 to 14, wherein the step of screening at least one microfluidic droplet for a first detectable signal comprises quantification the first detectable signal.
**Item 16:** The method according to any one of the preceding items, wherein the two or more reporter cells emit a second detectable signal, wherein the second detectable signal is independent of the first detectable signal, and preferably which is essentially independent of any biological, chemical or physical stimulus within the microfluidic droplet.
**Item 17:** The method according to any one of items 1 to 16, wherein the microfluidic droplet comprises a single antibody producing cell, such as a B-cell, and wherein the two or more reporter particles emit the first detectable signal upon an interaction with an antibody produced by said antibody producing cell.
**Item 18:** The method according to any one of items 1 to 17, wherein the detectable signal is fluorescent or luminescent signal.
**Item 19:** A microfluidic droplet, wherein the microfluidic droplet is a microfluidic droplet as defined according to the method of any one of items 1 to 18.
**Item 20:** A method for screening for an antigen binding construct having a biological activity, comprising a step of encapsulating each of a multiplicity of individual candidate antigen binding construct species, or each of a multiplicity of individual particles producing a candidate antigen binding construct species, into a microfluidic droplet in order to obtain a multiplicity of microfluidic droplets of which each comprises not more than one candidate antigen binding construct species, or not more than one individual particle producing a candidate antigen binding construct species, wherein the microfluidic droplet further comprises at least two reporter particles capable of emitting a first detectable signal representative for a biological activity (such as a signalling event), and wherein said at least two reporter particles emit said first detectable signal upon direct or indirect interaction with the candidate antigen binding construct.
**Item 21:** The method according to item 20, wherein the antigen binding construct is an antibody, or an antigen binding fragment thereof, or an antibody receptor, or an antigen binding fragment thereof, or a T-cell receptor (TCR), or an antigen binding fragment thereof, and a fusion protein comprising an antigen binding fragment of the aforesaid, such as a bispecific molecule or other multispecific molecule, a chimeric antigen receptor (CAR) etc.
**Item 22:** The method according to items 20 or 21, wherein if, upon direct or indirect interaction of the candidate antigen binding construct with the two or more reporter particles, the first detectable signal compared to a control is (i) increased, said candidate antigen binding construct has an agonistic biological activity, or is (ii) decreased, said candidate antigen binding construct has an antagonistic biological activity.
**Item 23**: The method according to any one of items 20 to 22, wherein the two or more reporter particles are biological cells.
**Item 24**: The method according to any one of items 20 to 23, wherein the microfluidic droplet comprises two to fifteen reporter particles, more preferably two to ten, more preferably two to eight, more preferably two to six, more preferably three to six.
**Item 25**: The method according to any one of items 20 to 24, wherein the two or more reporter particles are identical.
**Item 26**: The method according to item 25, wherein the at least one microfluidic droplet does not comprise any further reporter particle, the further reporter particle being characterized in that it is non-identical to the two or more reporter particles.
**Item 27**: The method according to any one of the preceding items, comprising a further step of sorting the microfluidic droplet according to the result of the screening step.

### BRIEF DESCRIPTION OF THE FIGURES

The figures show:

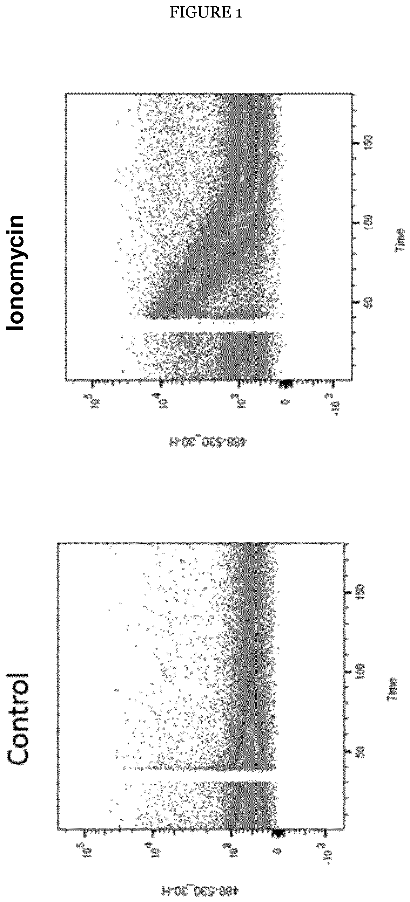

**Figure 1**: shows fluorescence signals of individual reporter cells loaded with a fluorescence probe for monitoring calcium flux, recorded in a flow cytometer. Cells were either untreated (left) or treated with Ionomycin (right), causing a strong fluorescence signal. Treatment control or Ionomycin were applied after about 40s (interrupted time series). The fluorescence intensity is plotted on the Y-axis, while time is shown on the X-axis. Each dot corresponds to the signal of one cell. Due to cell heterogeneity the fluorescence intensity varies significantly between different cells of the same type.

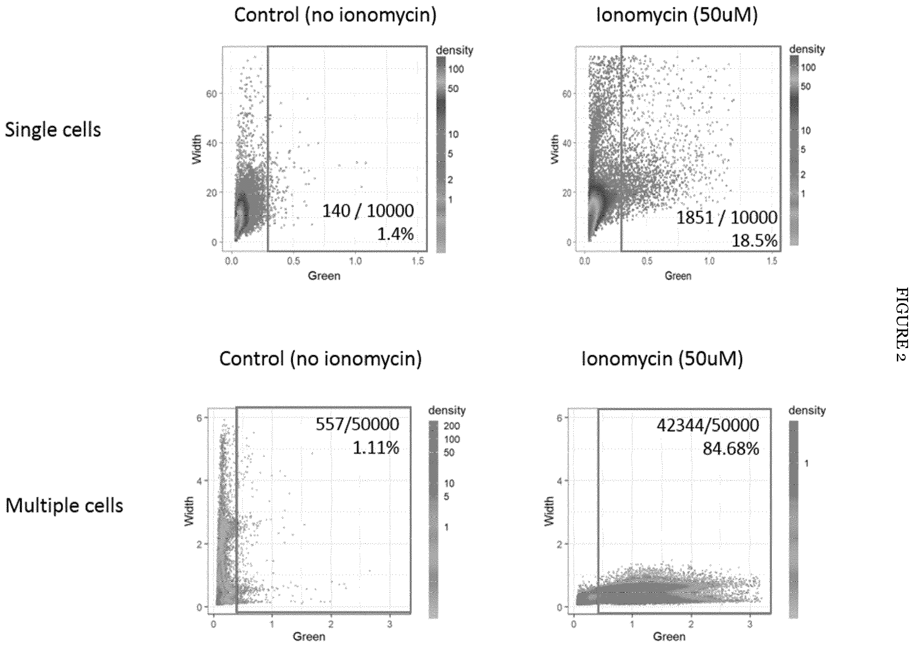

**Figure 2**: shows fluorescence signals of droplets hosting multiple reporter cells loaded with a fluorescence probe for monitoring calcium flux and stimulated with Ionomycin (causing Calcium flux). While due to biological variation and focusing issues only a fraction of the droplets show a positive signal (fluorescence in the green channel; X-axis) when having individual reporter cells in the droplet (upper panel; e.g. 18.5% in the desired gate), nearly all droplets show a positive reporter signal when encapsulating multiple reporter cells per droplet and plotting only the strongest fluorescence signal for each droplet.

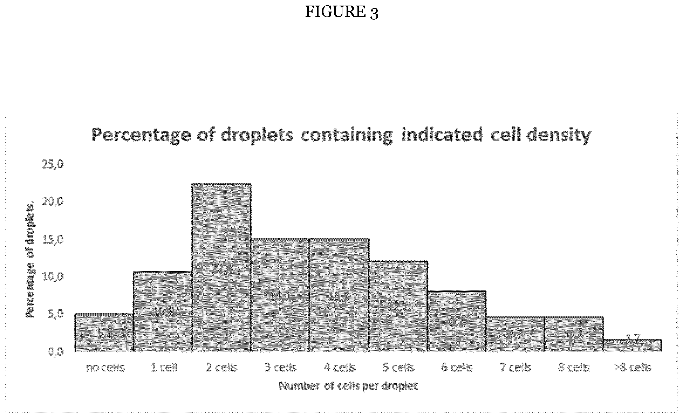

**Figure 3**: Poisson distribution of encapsulated reporter cells.

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### Example 1

One problem of microfluidic screening systems using encapsulated reporter cells is biological variation. Two cells of the same cell type can react differently, based on many biological parameters (cell size, cell cycle phase, stoichiometric variations in protein abundancy, etc.). This is particularly problematic when running single-cell assays with reporter cells generating a fluorescence signal upon a desired effect. For example, the stimulation of G-protein coupled receptors (GPCRs) on the surface of individually encapsulated cells can trigger a Ca-flux signal that can be assayed using fluorescent probes. For screening purposes one would like to have a scenario in which the same stimulation (e.g. concentration of an agonistic ligand) results in the same level of fluorescence.

However, due to biological variation, this is not the case, no matter if measuring the fluorescence of individual cells in a flow cytometer (figure 1) or in microfluidic droplets (for which the effect that cells can freely float inside the droplet, meaning at any point in time they can be either within or outside the focal plane has to be taken into account additionally; figure 2). In context of the invention the problem was overcome or at least it can be minimized by encapsulating more than one reporter cell into the microfluidic droplet and measuring/considering the strongest signal within the droplet (e.g. being generated by only one of the several co-encapsulated reporter cells; e.g. the one being mostly in the focal plane AND generating the highest number of reporter fluorophores), only. When doing so, the variation in the intensity of fluorescence signals generated by reporter cells that were stimulated with an agent causing calcium flux is surprisingly strongly decreased (figure 2).

In the present example, the majority of cells were encapsulated with 2 to 6 cells. It was not possible to sufficiently encapsulate more than 9 cells (figure 3).

## Claims

1. **A method of screening a plurality of microfluidic droplets with reduced signal heterogeneity,** comprising the step of screening at least one microfluidic droplet of the plurality of microfluidic droplets for the presence of a first detectable signal, wherein the at least one microfluidic droplet comprises particles and wherein the entirety of particles comprised in the microfluidic droplet consists of: (i) two to eight reporter particles capable of emitting the first detectable signal, and (ii) one single candidate signal inducing particle; wherein a direct or indirect interaction between the one single candidate signal inducing particle and the two or more reporter particles may induce emitting the first detectable signal by the two or more reporter particles.

2. The method according to claim 1, wherein the at least one microfluidic droplet comprises two to six, more preferably three to six.

3. The method according to claim 1 or 2, wherein each of the two or more reporter particles are identical.

4. The method according to claim 3, wherein the at least one microfluidic droplet does not comprise any further reporter particle, the further reporter particle being **characterized in that** it is substantially non-identical to the two or more reporter particles.

5. The method according to any one of claims 1 to 4, wherein a direct interaction is a binding between the one or more candidate signal inducing particle to the two or more reporter particles, and/or wherein an indirect interaction is a release of one or more compounds from the one or more candidate signal inducing particle, and wherein such compounds directly or indirectly interact with the two or more reporter particles.

6. The method according to any one of claims 1 to 5, wherein the two or more reporter particles are biological cells which emit the first detectable signal upon a biological, chemical or physical stimulus, preferably wherein the stimulus involves or induces any one or a combination of a protein-protein interaction, receptor-ligand interaction, molecule influx or efflux, etc.

7. The method according to claim 6, wherein the first detectable signal is emitted upon activation of an intracellular signal transduction pathway within the biological cell.

8. The method according to claim 11 or 12, wherein the biological cell comprises a signal probe, and said signal probe emits the first detectable signal upon said biological, chemical or physical stimulus.

9. The method according to any one of the preceding claims, wherein the two or more reporter cells emit a second detectable signal, wherein the second detectable signal is independent of the first detectable signal, and preferably which is essentially independent of any biological, chemical or physical stimulus within the microfluidic droplet induced by or caused by the candidate signal inducing particle.

10. The method according to any one of claims 1 to 9, wherein the microfluidic droplet comprises a single antibody producing cell, such as a B-cell, and wherein the two or more reporter particles emit the first detectable signal upon an interaction with an antibody produced by said antibody producing cell.

11. **A microfluidic droplet,** wherein the microfluidic droplet is a microfluidic droplet as defined according to the method of any one of claims 1 to 10.

12. **A method for screening for an antigen binding construct having a biological activity,** comprising a step of encapsulating each of a multiplicity of individual candidate antigen binding construct species, or each of a multiplicity of individual particles producing a candidate antigen binding construct species, into a microfluidic droplet in order to obtain a multiplicity of microfluidic droplets of which each comprises not more than one candidate antigen binding construct species, or not more than one individual particle producing a candidate antigen binding construct species, wherein the microfluidic droplet further comprises at least two reporter particles capable of emitting a first detectable signal representative for a biological activity (such as a signalling event), and wherein said at least two reporter particles emit said first detectable signal upon direct or indirect interaction with the candidate antigen binding construct.

13. The method according to claim 12, wherein the antigen binding construct is an antibody, or an antigen binding fragment thereof, or an antibody receptor, or an antigen binding fragment thereof, or a T-cell receptor (TCR), or an antigen binding fragment thereof, and a fusion protein comprising an antigen binding fragment of the aforesaid, such as a bispecific molecule or other multispecific molecule, a chimeric antigen receptor (CAR) etc.

14. The method according to claims 12 or 13, wherein if, upon direct or indirect interaction of the candidate antigen binding construct with the two or more reporter particles, the first detectable signal compared to a control is (i) increased, said candidate antigen binding construct has an agonistic biological activity, or is (ii) decreased, said candidate antigen binding construct has an antagonistic biological activity.

15. The method according to any one of the preceding claims, comprising a further step of sorting the microfluidic droplet according to the result of the screening step.
